# EUROPEAN PATENT APPLICATION

(11) **EP 0 551 846 A1**
(43) Date of publication of application: **21.07.1993**
(21) Application number: 93100270.3
(22) Date of filing: 11.01.1993
(51) Int. Cl.: A61B 17/58

(54) **Intramedullary pin for dynamic osteosynthesis in the femoral trochanteric region**

(30) Priority: 14.01.1992 IT BO920011
(71) Applicant: Zoli, Andrea, I-48010 Cotignola (Ravenna) (IT)
(72) Inventor: Zoli, Andrea, I-48010 Cotignola (Ravenna) (IT)
(74) Representative: Modiano, Guido, Dr.-Ing.

(57) **Abstract**

The intramedullary pin (1) has a proximal stub (2) transversed by at least one hole (3) with a transverse axis along which the stem (4) of a cervical spongiosa pin (6) or screw (5), rigidly coupleable to a neck-head of a damaged bone, is slidably longitudinally guided. The ends of curved and elastically deformable stems (11) having tips are axially rigidly coupled to the stub (2). The stems (11) are provided with a member for keeping the tips of the stems (11) mutually adjacent during the insertion of the pin (1) into a medullary canal through an apex of a greater trochanter, and for releasing the tips when the pin (1) is in a correct position in order to allow the stems (11) to elastically spread apart.

## Description

The present invention relates to an intramedullary pin for dynamic osteosynthesis with a self-locking distal end for fractures of the femoral trochanteric region.

In recent years, the concept of "biological" healing of fractures or elastic osteosynthesis has progressively become established. Two important factors determine such biological healing following surgical treatment, i.e., non-exposure of the fracture site and non-rigid or elastic internal fixation of the fracture itself. Micromovements, occuring between the bone segments which must heal and bond together, actually promote a more rapid healing of the fracture by promoting the formation of endosteal and periosteal callous.

In order to reduce fractures affecting the proximal part of the femur (for example for fractures of the trochanteric region of the femur) it is known to intervene with a pin or with plates which are anchored to the undamaged cortices of the long bone; the ends of one or more pins or screws for use in the cervical substantia spongiosa are slidably guided along one or more transverse axes with respect to said first pins or plates and are rigidly coupled to the neck and the head of the femur.

The spongiosa screws or pins are rigidly coupled, by means of their threaded ends, to the neck-head of the femur and while the fracture recomposes there is the possibility of relative sliding of the head along a transverse direction which is inclined with respect to the long bone, which facilitates and accelerates the healing process.

In order to fix the pin to the femoral diaphysis, it is known, in these operations, to use transverse screws which are screwed into the cortices: this fixing by means of screws entails some problems due, among other reasons, to the fact that additional surgical time is required and that it is usually necessary to resort to radiologic means for rather long periods in order to determine the fixing position of the screws or pins, with consequent exposure of the surgeon, assistants and operating-theater staff to harmful ionizing radiation.

In addition to this, when the pin must be removed at the end of the treatment, it is necessary to act not only in the bone region from which the nail must be removed but also distally, where the fixing screws have to be removed.

An intramedullary pin for metadiaphyseal fractures of long bones provided with a self-locking end is known from Published European Patent Application No. 0 401 650, and has yielded excellent results as regards the ease and speed with which the self-locking distal end is anchored.

The technical aim of the present invention is to provide an intramedullary pin for dynamic osteosynthesis of lateral (or trochanteric) fractures of the femoral neck which does not require external action for distal pin-to-bone fixing, allows elastic and dynamic osteosynthesis, can be installed very quickly while requiring an extremely low exposure to radiation, does not require the preliminary preparation and drilling of the medullary canal, and can even more easily be removed once the fracture has repaired.

Within the scope of this technical aim, an object of the present invention is to provide a pin which can be installed with an extremely simple set of instruments, has a reduced cost and adapts to various dimensional situations of the bone in which it is installed, so that a very small number of sizes is sufficient to meet any requirement.

Another object of the present invention is to achieve the above aim with a structure which is simple, relatively easy to produce in practice, safe in use and effective in operation as well as relatively modest in cost.

This aim and these objects are achieved by the present intramedullary pin for dynamic osteosynthesis with a self-locking end for fractures of the femoral trochanteric region, characterized in that it comprises a proximal stub crossed by at least one hole with a transverse axis along which the stem of a fixing member, rigidly coupleable to a neck-head of a damaged bone, is longitudinally slidably guided, the ends of at least two curved and elastically deformable stems being axially rigidly coupled to said stub, said stems having free ends provided with means for temporary mutual coupling, said means being suitable to keep the tips of the stems mutually adjacent during the insertion of the pin into a medullary canal through an apex of a greater trochanter and to release the tips when the pin is in a correct position in order to allow the stems to elastically spread apart.

The stems anchor, by means of their tips, in the intramedullary spongy bone to the point, in some instances, of pressing on the internal walls of the bone, producing a rigid coupling between the synthesis means and the bone.

Further peculiarities will become apparent and evident from the detailed description of a preferred but not exclusive embodiment of an intramedullary pin according to the invention, illustrated only by way of non-limitative example in the accompanying drawings, wherein:
figure 1 is a perspective view of an intramedullary pin according to the present invention after the release of the tips of the stems;
figure 2 is a side view of a pin according to the invention, in an installed condition;
figures 3 and 4 are views of two versions of the upper part of the nail of figure 2;
figure 5 is an enlarged perspective view of the means for the temporary coupling of the stems (pin closed and ready for insertion).

With particular reference to the above figures, the reference numeral 1 generally designates the intramedullary pin or nail according to the invention, for fractures of the trochanteric region of the femur.

The pin 1 is constituted by a proximal stub 2 which is crossed by at least one hole 3 whose axis is transverse to the stub and along which the stem 4 of a fixing member such as a cervical spongiosa screw 5 or pin 6 is slidably longitudinally guided; said screw or pin is rigidly coupled, for example, by screwing the self-threading portion 7, into the undamaged head of the femur.

Conveniently, the screw 5 is cannulated, i.e. axially crossed by a hole for a guiding wire which is arranged through the stub and the bone after drilling the bone with the drill and with a bit whose diameter is less than the diameter of the screw and is used to correctly guide the threaded end of the screw.

The stub 2 is slightly curved so as to assume a more correct anatomical shape and improve load distribution and avoid fractures of the femoral cortex.

At one end, the stub has an axial threaded hole 8 in which the stem 9 of a handle-like member 10 or grip instrument is screwed; said handle-like member facilitates the operations for inserting and removing the nail in and from the bone.

At least two elastically deformable curved stems 11 (advantageously made of biocompatible steel such as for example AISI 316 L), six in the particular embodiment shown, are rigidly coupled to the stub 2 at its other end and can advantageously have a circular cross-section and rounded ends.

Conveniently, the base of the stub has a slight truncated taper which converges toward the couplings of the stems.

Means for temporarily retaining the stems in a bundle are provided and consist, for example, of a metallic wire 12 which has a manual grip ring rigidly coupled to one of its ends; proximate to their ends, the stems have respective grooves 15 in which oval rings 16 are arranged; said rings are compressed in order to rigidly couple to the grooves, assuming essentially the shape of a figure eight, so as to define respective holes 17 in which the metallic wire 12 can be inserted; conveniently, the wire is passed longitudinally inside the upper stub.

The operation of the pin according to the invention is as follows:
the pin is inserted in the medullary canal from the apex of the greater trochanter; by using a radiographic investigation means, the pin is pushed up to the distal metadiaphysis of the femur; here the wire 12 is pulled, releasing the stems and allowing them to spread out; as the insertion of the pin continues, the stems, by spreading out, press the bone of the metadiaphysis M from the inside and become anchored to it.

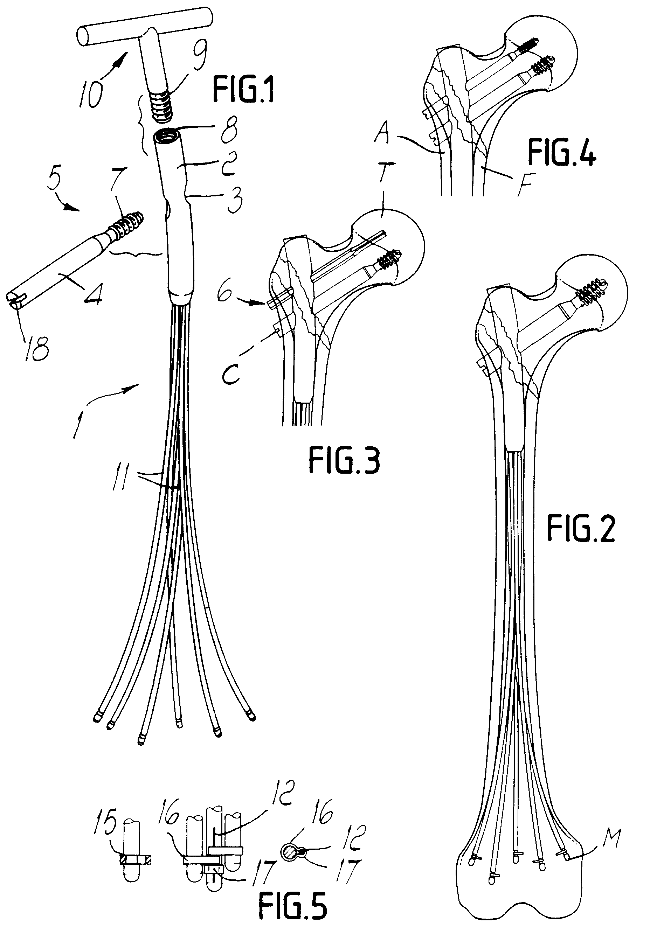

At this point it is possible to drill the bone of the metadiaphysis A and of the inside of the head T at an angle defined by an axis designated by the letter C (fig. 3), using alignment systems which are rigidly coupled to the stub of the pin. A guiding wire is inserted in said holes and keeps the holes provided in the femoral bone and the hole of the stub mutually aligned.

By using this guiding wire it is possible to insert the screw 4, which correctly centers all the holes, and then screw it by acting on the notch 18.

Attention is drawn to the fact that the self-locking end fixes to the distal part of the femur without having to operate on this region from the outside; furthermore, since the various stems become arranged on different planes, torsional movements of the stub with respect to the bone are prevented.

The sliding allowed between the pin or screw and the stub provides, between the ends of the fracture, a coupling which instead of being rigid is slightly elastic and can slide, which allows quicker formation of endosteal and periosteal callous.

Figures 2, 3 and 4 illustrate different possibilities of embodiment with one screw, a screw and a pin, and two parallel screws.

It is stressed that advantageously, only a few sizes of the pin according to the invention are sufficient to be able to operate on any type of patient and fracture and that no preliminary operations for drilling or preparing the medullary canal are required.

It has thus been observed that the invention achieves the intended aim and objects. The invention thus conceived is susceptible to numerous modifications and variations, all of which are within the scope of the inventive concept. All the details may furthermore be replaced with other technically equivalent ones. In practice, the materials employed, as well as the shapes and dimensions, may be any according to the requirements without thereby abandoning the protective scope of the following claims.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. Intramedullary pin for dynamic osteosynthesis with a self-locking distal end for fractures of the femoral trochanteric region, characterized in that it comprises a proximal stub crossed by at least one hole with a transverse axis along which the stem of a fixing member, rigidly coupled to the neck-head of the damaged bone, is slidably longitudinally guided, the ends of at least two curved and elastically deformable stems being axially rigidly coupled to said stub, said stems having free ends provided with means for temporary mutual coupling, said means being suitable to keep the tips of the stems mutually adjacent during the insertion of the pin into a medullary canal through an apex of a greater trochanter and to release the tips when the pin is in a correct position in order to allow the stems to elastically spread apart.

2. Pin according to claim 1, characterized in that said hole has an axis arranged on a plane which is substantially radial with respect to the axis of the stub.

3. Pin according to claim 1, characterized in that said means for the temporary mutual retention of the stems are constituted by rings which are compressed so as to rigidly couple inside corresponding peripheral grooves of the ends of the stems and define respective holes in which it is possible to insert a retention wire which extends along the stems and can be pulled out from the end of the stub in order to release the stems.

4. Pin according to claim 1, characterized in that an axial threaded hole for the screwing of a grip handle is provided on the head of the stub, said handle being suitable to facilitate the operations for the insertion and extraction of the pin into and from a medullary canal and to receive an alignment instrument for drilling the outer cortex of the femur.

5. Pin according to claim 1, characterized in that said stub is slightly curved and has a base with a slight converging taper.

6. Pin according to claim 3, characterized in that said stub is perforated axially for the passage of said metallic wire.
